# EUROPEAN PATENT APPLICATION

(11) **EP 4 711 363 A1**
(43) Date of publication of application: **18.03.2026**
(21) Application number: 24802907.6
(22) Date of filing: 06.05.2024
(51) Int. Cl.: C07D 413/06, C07D 207/08

(54) **PREPARATION METHOD FOR INTERMEDIATE OF AURISTATIN DERIVATIVE**

(30) Priority: 06.05.2023 CN 202310518856
(71) Applicant: RemeGen Co., Ltd., Shandong 264006 (CN)
(72) Inventor: WANG, Lixin, Beijing Middle Road, Yantai Development Zone, Yantai District, China (Shandong) Pilot Free Trade Zone Yantai, Shandong 264006 (CN); GAO, Jinwei, Beijing Middle Road, Yantai Development Zone, Yantai District, China (Shandong) Pilot Free Trade Zone Yantai, Shandong 264006 (CN); ZHONG, Cheng, Beijing Middle Road, Yantai Development Zone, Yantai District, China (Shandong) Pilot Free Trade Zone Yantai, Shandong 264006 (CN); TANG, Haizhou, Beijing Middle Road, Yantai Development Zone, Yantai District, China (Shandong) Pilot Free Trade Zone Yantai, Shandong 264006 (CN); YE, Hui, Beijing Middle Road, Yantai Development Zone, Yantai District, China (Shandong) Pilot Free Trade Zone Yantai, Shandong 264006 (CN); ZHAO, Hongyan, Beijing Middle Road, Yantai Development Zone, Yantai District, China (Shandong) Pilot Free Trade Zone Yantai, Shandong 264006 (CN); ZHU, Marie M., Beijing Middle Road, Yantai Development Zone, Yantai District, China (Shandong) Pilot Free Trade Zone Yantai, Shandong 264006 (CN)
(74) Representative: Maiwald GmbH
(86) International application number: PCT/CN2024/091109
(87) International publication number: WO 2024/230625

(57) **Abstract**

Provided in the present invention is a process route for preparing an intermediate of an auristatin derivative. Compared with the prior art, said process route effectively avoids using norephedrine in the synthesis path; and in addition, said process route provided in the present invention can produce a target product in one step, effectively avoiding generation of reaction intermediate products, and making a purification method simple and the yield and purity of a final product high. Therefore, the process route is suitable for industrial scale-up production.

## Description

### FIELD

The present disclosure relates to the field of pharmaceutical intermediate synthesis, in particular to a method for producing an auristatin derivative intermediate.

### BACKGROUND

An antibody-drug conjugate (ADC) is an anti-tumor drug, comprising 3 components: an antibody, a linker, and a toxin (drug), wherein the antibody and the toxin are conjugated via the linker. The mechanism of action of ADCs involves delivering the drug to target cells (such as tumor cells) utilizing the targeting ability of the antibody, where the drug is released to kill the tumor cells. At present, although a large quantity of natural or chemically synthesized cytotoxins are known, only a very small fraction of them can be applied in ADCs. It is mainly because toxins used as ADC payloads must possess a combination of characteristics, such as an extremely high cytotoxicity, an intracellular target, and a small molecular structure. Among these, auristatin compounds (such as MMAE and MMAF) are cytotoxic molecules that have been successfully used in the development of multiple ADC drugs.

MMAE (monomethyl auristatin E), a chemically synthesized auristatin derivative, can effectively inhibit mitosis by inhibiting tubulin polymerization. At present, market price of MMAE is very high, partly because currently known synthetic routes of MMAE are relatively complicated, involve multiple steps, often require extreme conditions such as ultra-low temperatures, and result in low yields. Additionally, some of the substrates are controlled substances involving norephedrine and the like.

At present, a common route for producing MMAE is as follows, wherein compound (I-1) is a common starting material (an auristatin derivative intermediate):

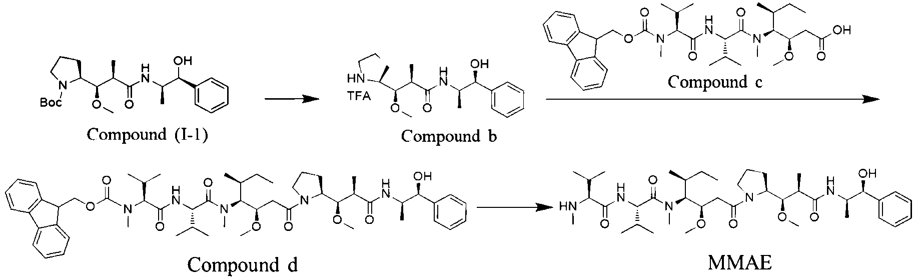

For producing the starting material compound (I-1), a preparation strategy is disclosed in Chinese patent publication No. CN107921144A, in which compound B is first produced from compound A (see Example 6 on page 49 of the specification), and is then reacted with different intermediates to produce auristatin derivatives with different substituent groups (see, for example, Example 11 on page 51, Example 17 on page 53, Example 25 on page 55 of the specification).

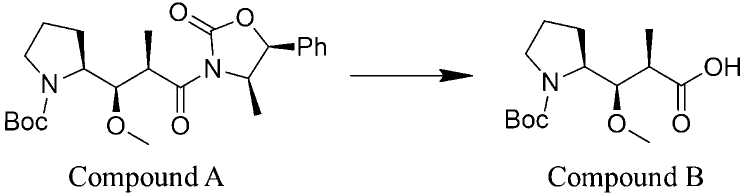

Page 130, line [1061] of the specification of Chinese patent publication No. CN104185477A discloses a process for producing compound (I-1) from compound B, via the following route:

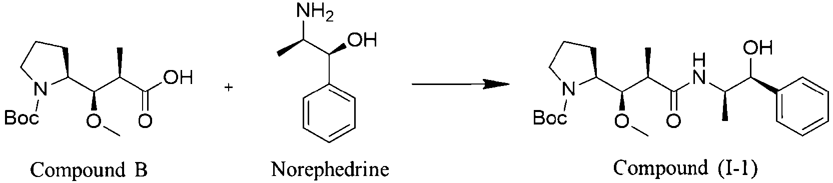

The aforementioned process for producing compound (I-1) is a two-step reaction (namely compound A → compound B → compound (I-1)). This process not only has technical deficiencies such as a reduced final product yield and an increased production costs, but also has a critical deficiency that a starting material used, norephedrine, is a precursor chemical which can be converted into methamphetamine, commonly known as "meth", through a relatively simple chemical transformation. Therefore, norephedrine is a controlled substance whose procurement is limited, which in turn increases the complexity and risk of the production process.

### SUMMARY

In view of the problems in the prior art, the present disclosure provides a novel process route for producing the aforementioned auristatin derivative intermediate. Specifically, the auristatin derivative intermediate in the present disclosure is a compound represented by formula (I), or an enantiomer, a racemate or a pharmaceutically acceptable salt thereof 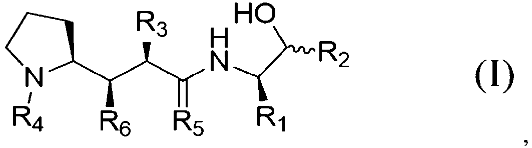 which is produced via a route of: 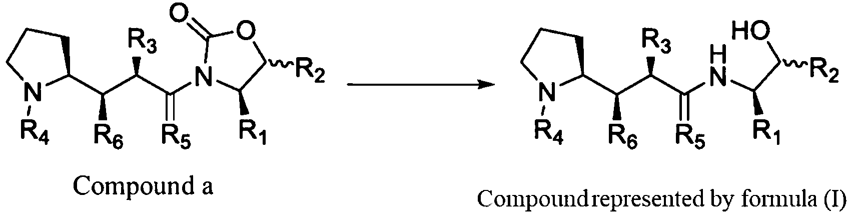 wherein:
R₁, R₂, and R₃ are independently selected from the group consisting of H, C₁-C₈ alkyl, C₁-C₈ heteroalkyl, C₂-C₈ alkenyl, C₂-C₈ alkynyl, C₃-C₈ heterocycle, C₃-C₈ carbocycle, aryl, C₁-C₈ alkaryl; C₁-C₈ alkyl carbocycle, and C₁-C₈ alkyl heterocycle;
R₄ is an amino protecting group;
R₅ is O or S;
R₆ is selected from the group consisting of H, -OH, C₁-C₈ alkyl, C₃-C₈ carbocycle, and -O-(C₁-C₈ alkyl).

The method for producing auristatin derivative intermediate provided by the present disclosure comprises steps of
step 1: dissolving compound a in an appropriate amount of organic solvent 1;
step 2: adding an aqueous solution of inorganic base to the solution obtained in step 1, and performing reaction; and
step 3: performing post-process on the reaction system obtained in step 2, and obtaining the compound represented by formula (I);
wherein the organic solvent 1 is one or more selected from the group consisting of N,N-dimethylformamide, N-methylpyrrolidone, methanol, ethanol, isopropanol, acetonitrile, acetone, tetrahydrofuran, and 1,4-dioxane.

In some preferred embodiments, the organic solvent 1 is methanol; in some other preferred embodiments, the organic solvent 1 is absolute ethanol; in some other preferred embodiments, the organic solvent 1 is tetrahydrofuran; in some other preferred embodiments, the organic solvent 1 is acetonitrile.

In some other preferred embodiments, the organic solvent 1 is a mixture of any two selected from the group consisting of N,N-dimethylformamide, N-methylpyrrolidone, methanol, ethanol, isopropanol, acetonitrile, acetone, tetrahydrofuran, and 1,4-dioxane, such as a mixture of methanol and ethanol in any volume ratio, a mixture of N,N-dimethylformamide and N-methylpyrrolidone in any volume ratio, a mixture of N-methylpyrrolidone and methanol in any volume ratio, a mixture of ethanol and isopropanol in any volume ratio, a mixture of isopropanol and acetonitrile in any volume ratio, a mixture of acetonitrile and acetone in any volume ratio, a mixture of acetone and tetrahydrofuran in any volume ratio, a mixture of tetrahydrofuran and 1,4-dioxane in any volume ratio, or a mixture of any other combination thereof in any volume ratio. When the organic solvent 1 is a mixture of two reagents, their volume ratio can be arbitrary, such as 1:1, 1:2, 1:3, 1:4..., or 2:1, 3:1, 4:1..., or any other volume ratio.

Preferably, R₁ is selected from the group consisting of H, C₁-C₈ alkyl, C₁-C₈ heteroalkyl, C₂-C₈ alkenyl, C₂-C₈ alkynyl, C₃-C₈ heterocycle, C₃-C₈ carbocycle, aryl, C₁-C₈ alkaryl; C₁-C₈ alkyl carbocycle, and C₁-C₈ alkyl heterocycle; preferably, R₁ is C₁-C₈ alkyl; more preferably, R₁ is selected from the group consisting of methyl, ethyl, propyl, butyl, pentyl, hexyl, heptyl, and octyl; more preferably, R₁ is selected from the group consisting of methyl and ethyl

Preferably, R₂ is selected from the group consisting of H, C₁-C₈ alkyl, C₁-C₈ heteroalkyl, C₂-C₈ alkenyl, C₂-C₈ alkynyl, C₃-C₈ heterocycle, C₃-C₈ carbocycle, aryl, C₁-C₈ alkaryl; C₁-C₈ alkyl carbocycle, and C₁-C₈ alkyl heterocycle; preferably, R₂ is aryl

Preferably, R₃ is selected from the group consisting of H, C₁-C₈ alkyl, C₁-C₈ heteroalkyl, C₂-C₈ alkenyl, C₂-C₈ alkynyl, C₃-C₈ heterocycle, C₃-C₈ carbocycle, aryl, C₁-C₈ alkaryl; C₁-C₈ alkyl carbocycle, and C₁-C₈ alkyl heterocycle; preferably, R₃ is C₁-C₈ alkyl; more preferably, R₃ is selected from the group consisting of methyl, ethyl, propyl, butyl, pentyl, hexyl, heptyl, and octyl; more preferably, R₃ is selected from the group consisting of methyl and ethyl

R₄ is selected from the group consisting of a Boc protecting group, a Cbz protecting group, a Fmoc protecting group, and a benzyl. In some specific embodiments, R₄ is a Boc protecting group; in some specific embodiments, R₄ is a Cbz protecting group; in some specific embodiments, R₄ is a Fmoc protecting group; in some other specific embodiments, R₄ is a benzyl

Preferably, R₅ is O; or preferably, R₅ is S.

Preferably, R₆ is selected from the group consisting of H, -OH, C₁-C₈ alkyl, C₃-C₈ carbocycle, and -O-(C₁-C₈ alkyl); more preferably, R₆ is -O-(C₁-C₈ alkyl); even more preferably, R₆ is methoxy.

Preferably, the compound a has a structure selected from the group consisting of

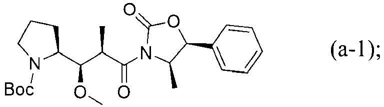

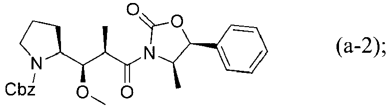

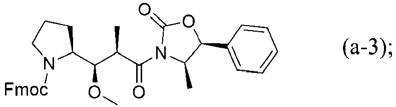

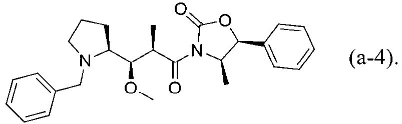

Preferably, the compound represented by formula (I) has a structure selected from the group consisting of

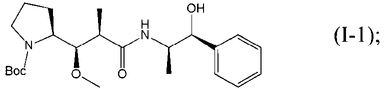

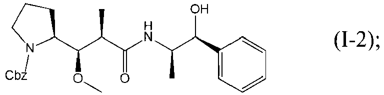

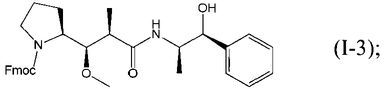

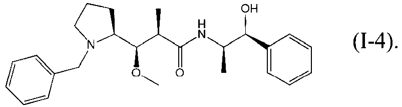

The inorganic base in step 2 is selected from the group consisting of lithium hydroxide, sodium hydroxide, potassium hydroxide, calcium hydroxide, potassium carbonate, cesium carbonate, and sodium carbonate; in some preferred embodiments, the inorganic base in step 2 is lithium hydroxide; in some other preferred embodiments, the inorganic base in step 2 is sodium hydroxide; in some other preferred embodiments, the inorganic base in step 2 is potassium hydroxide; in some other preferred embodiments, the inorganic base in step 2 is calcium hydroxide; in some other preferred embodiments, the inorganic base in step 2 is potassium carbonate; in some other preferred embodiments, the inorganic base in step 2 is cesium carbonate; in some other preferred embodiments, the inorganic base in step 2 is sodium carbonate.

In some specific embodiments, the aqueous solution of inorganic base has a mass fraction of 2% to 15%; preferably, the aqueous solution of inorganic base has a mass fraction of3% to 10%. In some preferred embodiments, the aqueous solution of inorganic base has a mass fraction of 2%; in some preferred embodiments, the aqueous solution of inorganic base has a mass fraction of 3%; in some preferred embodiments, the aqueous solution of inorganic base has a mass fraction of 4%; in some preferred embodiments, the aqueous solution of inorganic base has a mass fraction of 5%; in some other preferred embodiments, the aqueous solution of inorganic base has a mass fraction of 6%; in some other preferred embodiments, the aqueous solution of inorganic base has a mass fraction of 7%; in some other preferred embodiments, the aqueous solution of inorganic base has a mass fraction of 8%; in some other preferred embodiments, the aqueous solution of inorganic base has a mass fraction of 9%; in some other preferred embodiments, the aqueous solution of inorganic base has a mass fraction of 10%; in some other preferred embodiments, the aqueous solution of inorganic base has a mass fraction of 11%; in some other preferred embodiments, the aqueous solution of inorganic base has a mass fraction of 12%; in some other preferred embodiments, the aqueous solution of inorganic base has a mass fraction of 13%; in some other preferred embodiments, the aqueous solution of inorganic base has a mass fraction of 14%; in some other preferred embodiments, the aqueous solution of inorganic base has a mass fraction of 15%. In some other preferred embodiments, the aqueous solution of inorganic base can also be of other mass fractions, such as 4.8%, 5.5%, 5.7%, 7.4%, 9.4%, 8.9%, 9.1%, and the like.

In some other specific embodiments, the compound a and the organic solvent 1 have a weight-to-volume ratio (g/mL) of W_{compomd a}:V_{organic solvent 1} being 1:3 to 1:30. Preferably, the compound a and the organic solvent 1 have a weight-to-volume ratio (g/mL) of W_{compound a}:V_{organic solvent 1} being 1:3 to 1:14. In some preferred embodiments, the compound a and the organic solvent 1 have a weight-to-volume ratio (g/mL) of W_{compound a}:V_{organic solvent 1} being 1:3; in some other preferred embodiments, the compound a and the organic solvent 1 have a weight-to-volume ratio (g/mL) of W_{compound a}:V_{organic solvent 1} being 1:4, 1:5, 1:6, 1:7, 1:8, 1:9, 1:10, 1:11, 1:12, 1:13, 1:14, 1:15, 1:16, 1:17, 1:18, 1:19, 1:20, 121, 122, 123, 1:24, 125, 126, 1:27, 1:28, 1:29, or 1:30, or other non-integer ratios, such as about 1:3.1, ..., 1:3.2, ..., 1:5.27, ..., 1:6.23, ..., 1:6.72, ..., 1:10.32, ..., 1:11.21, ..., 1:13.45, and the like.

In some other specific embodiments, the compound a and the inorganic base have a molar ratio of 1:1 to 1:10. Preferably, the compound a and the inorganic base have a molar ratio of 1:1 to 1:5. In some preferred embodiments, the compound a and the inorganic base have a molar ratio of 1:1; in some preferred embodiments, the compound a and the inorganic base have a molar ratio of 1:2, 1:3, 1:4, 1:5, 1:6, 1:7, 1:8, 1:9 or 1:10. It should be understood that, in some other preferred embodiments, the compound a and the inorganic base can also have a non-integer molar ratio, such as 1:1.1, ..., 1:1.5, ..., 12.6, ..., 1:4.8, and the like.

In some other specific embodiments, the reaction in step 2 is performed at a temperature of -10 to 78°C; preferably, the reaction in step 2 is performed at a temperature of 5 to 40°C; more preferably, the reaction in step 2 is performed at a temperature of 15 to 35°C (such as 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C); in some preferred embodiments, the reaction in step 2 can be performed within a temperature range, such as 20±5°C, ..., 30±5°C, and the like.

Furthermore, in step 3, the post-process is performed after the reaction system obtained in step 2 is adjusted to be acidic.

Further preferably, in step 3, the post-process is performed after the reaction system obtained in step 2 is adjusted to pH=1 to 6, such as pH=1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0. It can also be other values such as 1.15, 2.26, 3.48, and the like.

Furthermore, the post-process in step 3 mainly comprises an extraction process and a crystallization process.

Furthermore, an extraction solvent used in the extraction process is one or more selected from the group consisting of ethyl acetate, dichloromethane, methyl tert-butyl ether, isopropyl acetate, and 2-methyltetrahydrofuran.

It should be understood that, the volume of the extraction solvent should not be regarded as a limitation of the present disclosure. In some preferred embodiments, the compound a and the extraction solvent have a weight-to-volume ratio (g/mL) of W_{compound a}:V_{extraction solvent} being 1:10 to 1:30 (for example 1:10, 1:11, 1:12, 1:13, 1:14, 1:15, 1:16, 1:17, 1:18, 1:19, 1:20, 121, 1:22, 1:23, 1:24, 1:25, 1:26, 127, 128, 1:29, 1:30, or other ratios, not restrictively, such as 1:14.55, 1:14.57, 1:19.49, 120.17, 1:24.66, and the like).

Furthermore, a crystallization solvent used in the crystallization process is one or more selected from the group consisting of N,N-dimethylformamide, N-methylpyrrolidone, methanol, ethanol, isopropanol, acetonitrile, acetone, 1,4-dioxane, ethyl acetate, dichloromethane, isopropyl acetate, tetrahydrofuran, 2-methyltetrahydrofuran, methyl tert-butyl ether, n-hexane, n-heptane, cyclohexane, and methylcyclopentane.

In some preferred embodiments, the crystallization solvent is one selected from the group consisting of N,N-dimethylformamide, N-methylpyrrolidone, methanol, ethanol, isopropanol, acetonitrile, acetone, 1,4-dioxane, ethyl acetate, dichloromethane, isopropyl acetate, tetrahydrofuran, 2-methyltetrahydrofuran, methyl tert-butyl ether, n-hexane, n-heptane, cyclohexane, and methylcyclopentane.

In some other preferred embodiments, the crystallization solvent is a mixture of two (namely crystallization solvent 1 and crystallization solvent 2) selected from the group consisting of N,N-dimethylformamide, N-methylpyrrolidone, methanol, ethanol, isopropanol, acetonitrile, acetone, 1,4-dioxane, ethyl acetate, dichloromethane, isopropyl acetate, tetrahydrofuran, 2-methyltetrahydrofuran, methyl tert-butyl ether, n-hexane, n-heptane, cyclohexane, and methylcyclopentane, for example, ethyl acetate/n-hexane, methyl tert-butyl ether/n-heptane, ethyl acetate/n-heptane, ..., and the like. Among them, the crystallization solvent 1 and crystallization solvent 2 can have any volume ratio, preferably 1:1, 1:2, 2:1, 1:3, 3:1, 1:4, 4:1, 1:5, 5:1, and the like.

It should be understood that, the volume of the crystallization solvent should not be regarded as a limitation of the present disclosure. In some preferred embodiments, the compound a and total volume of the crystallization solvent have a weight-to-volume ratio (g/mL) of W_{compounda}:V_{crystallization solvent} being 1:3 to 1:10 (for example 1:3, 1:4, 1:5, 1:6, 1:7, 1:8, 1:9, 1:10, or other ratios, not restrictively, such as about 1:3.11, 1:3.36, 1:5.27, 1:5.6, 1:8.97, 1:9.17, and the like).

Furthermore, the crystallization process is performed at a target temperature (namely the lowest temperature to which the crystallization process is cooled) of -25 to 60°C; preferably, the crystallization process is performed at a target temperature of -10 to 40°C; more preferably, the crystallization process is performed at a target temperature of 0 to 10°C. The target temperature can be a specific temperature or a range (for example 0°C, 2°C, 3°C, 4°C, 5°C, 6°C, 7°C, 8°C, 9°C, 10°C, 2±2°C, 3±2°C, 4±2°C, 5±2°C, 6±2°C, 7±2°C, 8±2°C, and the like).

It should be understood that, the aforementioned crystallization process can be carried out by first adding the crystallization solvent and then cooling the entire system to the target temperature. Alternatively, it can be carried out by first cooling the entire system to the target temperature and then adding the crystallization solvent.

Furthermore, the post-process in step 3 can comprise other processes in addition to the extraction process and the crystallization process, such as drying of the extract liquid, filtration and rotary evaporation of the extract liquid, filtration of the crystallization system, and other conventional operations.

Compared with the prior art, the process route for producing auristatin derivative intermediate provided by the present disclosure, effectively avoids the use of a controlled compound norephedrine, and can be performed under mild conditions. In addition, the process route for producing auristatin derivative intermediate provided by the present disclosure, can produce target product in a single step, which effectively prevents generation of reaction intermediates. The purification method is simple, and the final product exhibits a high yield and purity, making the process suitable for industrial scale-up production.

### DETAILED DESCRIPTION

### [Definitions]

The term "alkyl", as used in the present disclosure, refers to saturated aliphatic hydrocarbon groups, which are straight-chain or branched-chain groups containing 1 to 20 carbon atoms, preferably alkyls containing 1 to 12 carbon atoms, more preferably alkyls containing 1 to 10 carbon atoms, most preferably alkyls containing 1 to 8 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. The alkyl group may be substituted or unsubstituted. When alkyl is substituted, the substituents may be at any available point of attachment and are preferably one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, and oxo.

The term "heteroalkyl", as used in the present disclosure, refers to an alkyl group as defined above containing one or more heteroatoms selected from the group consisting of N, O, and S.

The term "alkenyl", as used in the present disclosure, refers to a straight-chain or branched-chain aliphatic hydrocarbon having one carbon-carbon double bond and 2 to 8 carbon atoms. Examples of alkenyl include: vinyl, allyl, n-butenyl, isobutenyl, 3-methylbut-2-enyl, n-pentenyl, hexenyl, heptenyl, and octenyl.

The term "alkynyl" as used in the present disclosure, refers to a straight-chain or branched-chain aliphatic hydrocarbon having one carbon-carbon triple bond and 2 to 8 carbon atoms. Examples of alkynyl include: ethynyl, propynyl, n-butynyl, 2-butynyl, 3-methylbutynyl, n-pentynyl, hexynyl, heptynyl, and octynyl.

The term "heterocycle", also referred to as "heterocyclyl", as used in the present disclosure, refers to a saturated or partially unsaturated monocyclic or polycyclic hydrocarbon substituent comprising 3 to 20 ring atoms, wherein one or more of the ring atoms are heteroatoms selected from the group consisting of nitrogen, oxygen, and S(O)u (where u is an integer from 0 to 2), preferably heterocycle or heterocyclyl comprises 3 to 8 ring atoms. Non-limiting examples of monocyclic heterocyclyl groups include pyrrolidinyl, piperidinyl, piperazinyl, morpholinyl, thiomorpholinyl and homopiperazinyl. Polycyclic heterocyclyl groups include spiro-, fused-, and bridged-heterocyclyl. The heterocyclyl may be optionally substituted or unsubstituted. When the heterocyclyl is substituted, the substituents are preferably one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, and oxo.

The term "aryl" as used in the present disclosure, refers to a 6- to 14-membered all-carbon monocyclic or fused-polycyclic (namely rings that share adjacent pairs of carbon atoms) group having a conjugated π-electron system, preferably having 6 to 10 members, for example, phenyl and naphthyl, preferably phenyl. Additionally, the aryl ring may be fused to a heteroaryl, heterocyclyl, or cycloalkyl ring, wherein the ring attached to the parent structure is the aryl ring. Non-limiting examples thereof include: 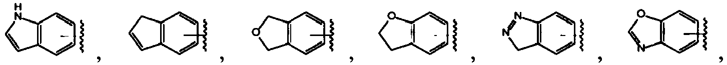 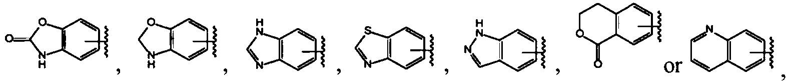 and the like.

The aryl may be substituted or unsubstituted. When the aryl is substituted, the substituents are preferably one or more groups independently selected from the group consisting of alkyl, alkenyl, alkynyl, alkoxy, alkylthio, alkylamino, halogen, hydroxyl, nitro, cyano, cycloalkyl, heterocyclyl, aryl, heteroaryl, cycloalkoxy, heterocycloalkoxy, cycloalkylthio, and heterocycloalkylthio.

The term "amino protecting group" as used in the present disclosure, refers to a group that protects an amino group from reacting while other parts of the molecule are undergoing a reaction, and can be easily removed. Non-limiting examples include 9-fluorenylmethoxycarbonyl (namely the Fmoc protecting group), tert-butoxycarbonyl (namely the Boc protecting group), benzyloxycarbonyl (namely the Cbz protecting group), acetyl, benzyl, allyl, p-methoxybenzyl, and the like. These groups may be optionally substituted with 1 to 3 substituents selected from the group consisting of halogen, alkoxy, and nitro.

The term "antibody drug conjugate" as used in the present disclosure, refers to a compound obtained by linking an antibody/functional fragment thereof, a linker, and a toxin (drug) through chemical reactions. Its structure is typically composed of three parts: an antibody or antibody-like ligand, a toxin part, and a linker that conjugates the antibody or antibody-like ligand and the drug part. The terms "drug" and "toxin moiety" as used herein, broadly refer to any compound that has a desired biological activity and has a reactive functional group for preparing the conjugates of the present disclosure.

### [Specific examples]

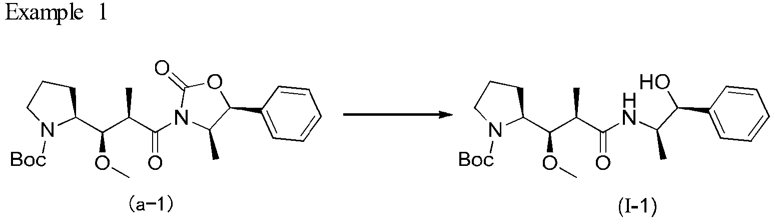

Compound (a-1) (4.46 g, 10 mmol) was dissolved in 14 mL of ethanol. Under nitrogen protection, an aqueous solution (24 mL, volume of water, the same applies hereinafter) of lithium hydroxide (1.2 g, 50 mmol) was added dropwise. After the addition was completed, the mixture was stirred at room temperature (20 to 25°C) for 30 min, then adjusted to be acidic (pH=5), and extracted with 65 mL of ethyl acetate. The ethyl acetate extract liquid was collected, dried over anhydrous Na₂SO₄, concentrated under reduced pressure to a certain volume, added with 25 mL of ethyl acetate/n-hexane (with a volume ratio of 1:5) and stirred. A solid gradually precipitated out. The mixture was cooled to 0°C under stirring and then filtered to obtain 3.15 g of a white solid, namely compound (I-1) (with a yield of 75% and a purity of 99.5%).

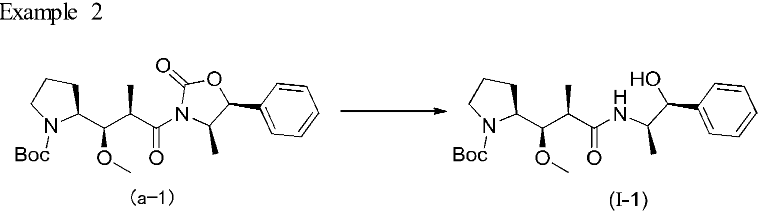

Compound (a-1) (4.46 g, 10 mmol) was dissolved in 14 mL of methanol. Under nitrogen protection, an aqueous solution (6 mL) of lithium hydroxide (0.48 g, 20 mmol) was added dropwise. After the addition was completed, the mixture was stirred at 15 °C for 30 min, then adjusted to be acidic (pH=4), and extracted with 90 mL of ethyl acetate. The ethyl acetate extract liquid was collected, dried over anhydrous Na₂SO₄, concentrated under reduced pressure to a certain volume, added with 25 mL of ethyl acetate/n-hexane (with a volume ratio of 1:5) and stirred. A solid gradually precipitated out. The mixture was cooled to 0°C under stirring and then filtered to obtain 3.32 g of a white solid, namely compound (I-1) (with a yield of 79% and a purity of 99.4%).

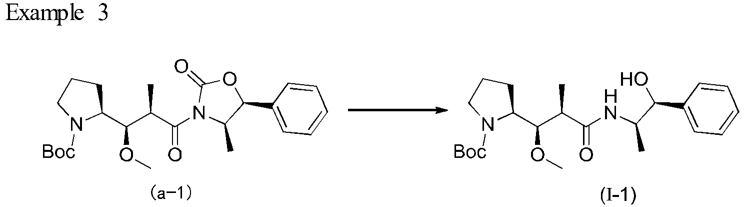

Compound (a-1) (4.46 g, 10 mmol) was dissolved in 60 mL of ethanol. Under nitrogen protection, an aqueous solution (10 mL) of sodium hydroxide (1.04 g, 26 mmol) was added dropwise. After the addition was completed, the mixture was stirred at 35 °C for 30 min, then adjusted to be acidic (pH=3), and extracted with 65 mL of dichloromethane. The dichloromethane extract was collected, dried over anhydrous Na₂SO₄, concentrated under reduced pressure to a certain volume, added with 40 mL of methyl tert-butyl ether and stirred. A solid gradually precipitated out. The mixture was cooled to 10°C under stirring and then filtered to obtain 3.15 g of a white solid, namely compound (I-1) (with a yield of 75% and a purity of 99.5%).

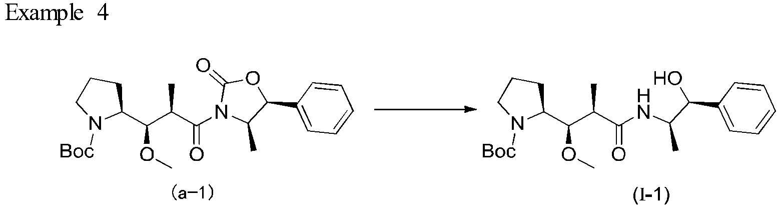

Compound (a-1) (4.46 g, 10 mmol) was dissolved in 50 mL of methanol. Under nitrogen protection, an aqueous solution (4.5 mL) of sodium hydroxide (0.44 g, 11 mmol) was added dropwise. After the addition was completed, the mixture was stirred at 35 °C for 30 min, then adjusted to be acidic (pH=2), and extracted with 90 mL of dichloromethane. The dichloromethane extract liquid was collected, dried over anhydrous Na₂SO₄, concentrated under reduced pressure to a certain volume, added with 15 mL of methyl tert-butyl ether/n-heptane (with a volume ratio of 2:1) and stirred. A solid gradually precipitated out. The mixture was cooled to 0°C under stirring and then filtered to obtain 3.57 g of a white solid, namely compound (I-1) (with a yield of 85% and a purity of 99.1%).

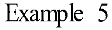

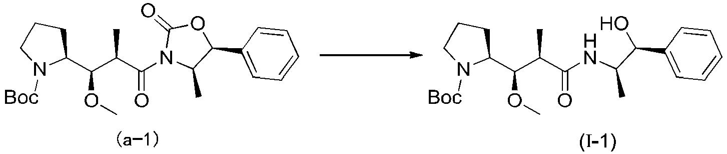

Compound (a-1) (4.46 g, 10 mmol) was dissolved in 30 mL of ethanol. Under nitrogen protection, an aqueous solution (12 mL) of lithium hydroxide (1.20 g, 50 mmol) was added dropwise. After the addition was completed, the mixture was stirred at room temperature (20 to 25°C) for 30 min, then adjusted to be acidic (pH=1), and extracted with 110 mL of ethyl acetate. The ethyl acetate extract liquid was collected, dried over anhydrous Na₂SO₄, concentrated under reduced pressure to a certain volume, added with 15 mL of ethyl acetate/n-heptane (with a volume ratio of 1:2) and stirred. A solid gradually precipitated out. The mixture was cooled to 10°C under stirring and then filtered to obtain 3.07 g of a white solid, namely compound (I-1) (with a yield of 73% and a purity of 99.3%).

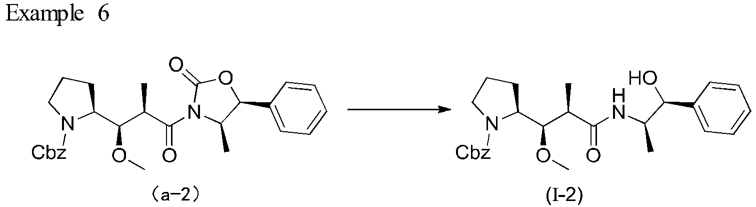

Compound (a-2) (4.81 g, 10 mmol) was dissolved in 30 mL of methanol. Under nitrogen protection, an aqueous solution (20 mL) of lithium hydroxide (1.20 g, 50 mmol) was added dropwise. After the addition was completed, the mixture was stirred at room temperature (20 to 25°C) for 30 min, then adjusted to be acidic (pH=6), and extracted with 70 mL of ethyl acetate. The ethyl acetate extract liquid was collected, dried over anhydrous Na₂SO₄, concentrated under reduced pressure to a certain volume, added with 15 mL of ethyl acetate/n-heptane (with a volume ratio of 1:2) and stirred. A solid gradually precipitated out. The mixture was cooled to 0°C under stirring and then filtered to obtain 3.80 g of a white solid, namely compound (I-2) (with a yield of 84% and a purity of 99.5%).

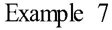

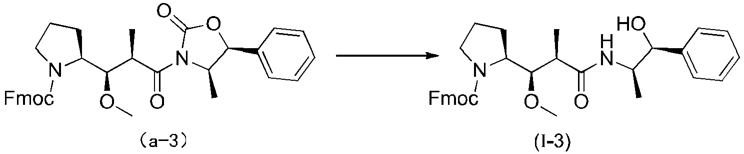

Compound (a-3) (5.69 g, 10 mmol) was dissolved in 30 mL of ethanol. Under nitrogen protection, an aqueous solution (4.5 mL) of lithium hydroxide (0.26 g, 11 mmol) was added dropwise. After the addition was completed, the mixture was stirred at room temperature (20 to 25°C) for 30 min, then adjusted to be acidic (pH=5.5), and extracted with 140 mL of dichloromethane. The dichloromethane extract was collected, dried over anhydrous Na₂SO₄, concentrated under reduced pressure to a certain volume, added with 30 mL of methyl tert-butyl ether and stirred. A solid gradually precipitated out. The mixture was cooled to 10°C under stirring and then filtered to obtain 1.95 g of a white solid, namely compound (I-3) (with a yield of 36% and a purity of 99.2%).

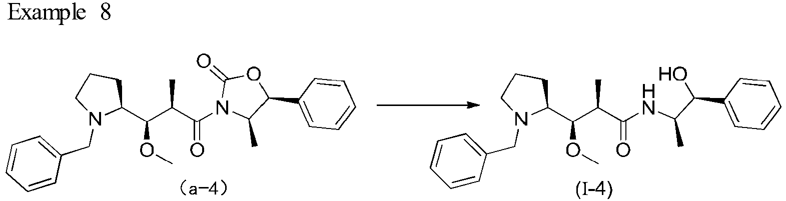

Compound (a-4) (4.36 g, 10 mmol) was dissolved in 45 mL of ethanol. Under nitrogen protection, an aqueous solution (4.5 mL) of sodium hydroxide (0.44 g, 11 mmol) was added dropwise. After the addition was completed, the mixture was stirred at room temperature (20 to 25°C) for 30 min, then adjusted to be acidic (pH=4.5), and extracted with 85 mL of ethyl acetate. The ethyl acetate extract liquid was collected, dried over anhydrous Na₂SO₄, concentrated under reduced pressure to a certain volume, added with 40 mL of ethyl acetate/n-heptane (with a volume ratio of 1:2) and stirred. A solid gradually precipitated out. The mixture was cooled to 0°C under stirring and then filtered to obtain 3.20 g of a white solid, namely compound (I-4) (with a yield of 78% and a purity of 99.6%).

The aforementioned description is only some preferred embodiments, which are only illustrated as examples and not to limit the combinations of features necessary to practice the present disclosure. The headings provided are not meant to limit the various embodiments of the present disclosure. Terms such as "comprise", "have" and "include" are not intended to be limiting. Furthermore, unless otherwise specified, the singular includes the plural, and the term "or" is used to mean "and/or". Unless otherwise defined herein, all technical and scientific terms used herein have the same meaning as commonly understood by those ordinarily skilled in the art.

All publications and patents mentioned in this application are incorporated herein by reference in their entirety. Various modifications and variations of the described methods and compositions of the present disclosure will be apparent to those skilled in the art without departing from the scope and spirit of the present disclosure. Although the present disclosure has been described in conjunction with specific preferred embodiments, it should be understood that the present disclosure as claimed should not be unduly limited to such specific embodiments. Indeed, various modifications of the described modes for carrying out the present disclosure, which are obvious to those skilled in the art, are intended to be included within the scope of the appended claims.

## Claims

1. A method for producing an intermediate of auristatin derivative, wherein the intermediate of auristatin derivative is a compound represented by formula (I) or an enantiomer, a racemate or a pharmaceutically acceptable salt thereof: 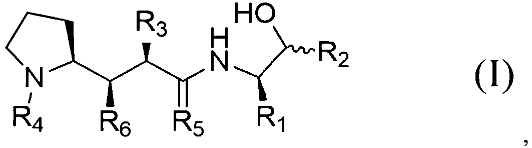 which is produced via a route of: 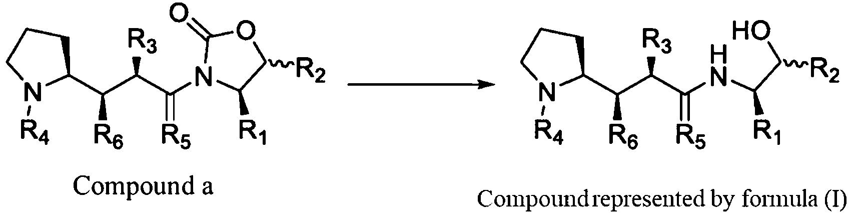 wherein:
R₁, R₂, and R₃ are independently selected from the group consisting of H, C₁-C₈ alkyl, C₁-C₈ heteroalkyl, C₂-C₈ alkenyl, C₂-C₈ alkynyl, C₃-C₈ heterocycle, C₃-C₈ carbocycle, aryl, C₁-C₈ alkaryl; C₁-C₈ alkyl carbocycle, and C₁-C₈ alkyl heterocycle;
R₄ is an amino protecting group;
R₅ is O or S;
R₆ is selected from the group consisting of H, -OH, C₁-C₈ alkyl, C₃-C₈ carbocycle, and -O-(C₁-C₈ alkyl); and
the method comprises steps of:
step 1: dissolving compound a in an appropriate amount of organic solvent 1;
step 2: adding an aqueous solution of inorganic base to the solution obtained in step 1, and performing reaction; and
step 3: performing post-process on the reaction system obtained in step 2, and obtaining the compound represented by formula (I);
wherein the organic solvent 1 is one or more selected from the group consisting of N,N-dimethylformamide, N-methylpyrrolidone, methanol, ethanol, isopropanol, acetonitrile, acetone, tetrahydrofuran and 1,4-dioxane.

2. The method according to claim 1, wherein the R₄ is selected from the group consisting of a Boc protecting group, a Cbz protecting group, a Fmoc protecting group, and a benzyl

3. The method according to claim 1, wherein the compound a has a structure selected from the group consisting of

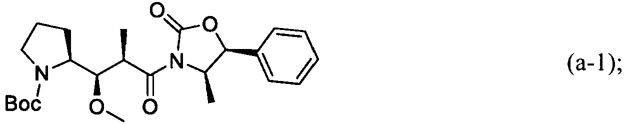

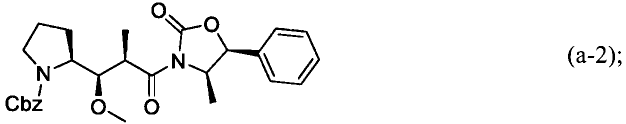

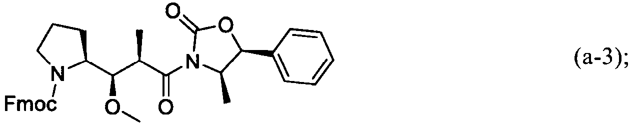

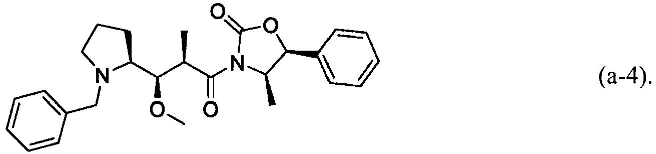

4. The method according to claim 1, wherein the compound represented by formula (I) has a structure selected from the group consisting of:

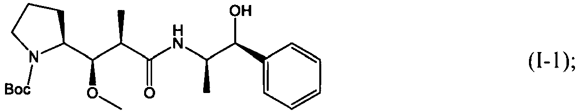

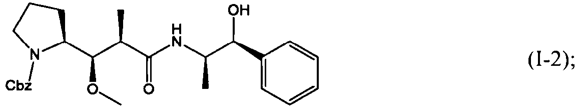

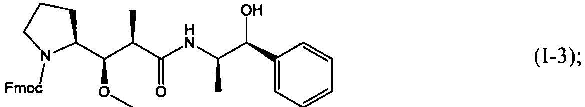

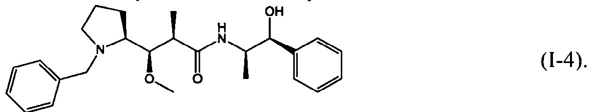

5. The method according to claim 1, wherein the organic solvent 1 is one selected from the group consisting of methanol and ethanol.

6. The method according to claim 1, wherein the inorganic base in step 2 is selected from the group consisting of lithium hydroxide, sodium hydroxide, potassium hydroxide, calcium hydroxide, potassium carbonate, cesium carbonate and sodium carbonate.

7. The method according to claim 1, wherein the aqueous solution of inorganic base has a mass fraction of 2% to 15%; preferably, the aqueous solution of inorganic base has a mass fraction of 3% to 10%; preferably, the compound a and the organic solvent 1 have a weight-to-volume ratio (g/mL) of W_{compound a}:V_{organic solvent} 1 being 1:3 to 1:30; further preferably, the compound a and the organic solvent 1 have a weight-to-volume ratio (g/mL) of W_{compound a}:V_{organic solvent 1} being 1:3 to 1:14.

8. The method according to claim 1, wherein the compound a and the inorganic base have a molar ratio of 1:1 to 1:10; preferably, the compound a and the inorganic base have a molar ratio of 1:1 to 1:5.

9. The method according to claim 1, wherein the reaction in step 2 is performed at a temperature of -10 to 78°C; preferably, the reaction is performed at a temperature of 5 to 40°C; preferably, the reaction is performed at a temperature of 15 to 35°C.

10. The method according to claim 1, wherein in step 3, the post-process is performed after the reaction system obtained in step 2 is adjusted to be acidic.

11. The method according to claim 1, wherein the post-process in step 3 comprises an extraction process and a crystallization process; preferably, an extraction solvent used in the extraction process is one or more selected from the group consisting of ethyl acetate, dichloromethane, methyl tert-butyl ether, isopropyl acetate and 2-methyltetrahydrofuran; preferably, a crystallization solvent used in the crystallization process is one or more selected from the group consisting of N,N-dimethylformamide, N-methylpyrrolidone, methanol, ethanol, isopropanol, acetonitrile, acetone, 1,4-dioxane, ethyl acetate, dichloromethane, isopropyl acetate, tetrahydrofuran, 2-methyltetrahydrofuran, methyl tert-butyl ether, n-hexane, n-heptane, cyclohexane and methylcyclopentane.

12. The method according to claim 11, wherein the crystallization process is performed at a target temperature of -25 to 60°C; preferably, the crystallization process is performed at a target temperature of -10 to 40°C; more preferably, the crystallization process is performed at a target temperature of 0 to 10°C.
